(19) Europäisches Patentamt / European Patent Office / Office européen des brevets

(11) **EP 4 412 755 B1**

(12) **FASCICULE DE BREVET EUROPEEN**

(45) Date de publication et mention
de la délivrance du brevet:
**24.12.2025 Bulletin 2025/52**

(21) Numéro de dépôt: **22797424.3**

(22) Date de dépôt: **03.10.2022**

(51) Classification Internationale des Brevets (IPC):
**B01J 8/02** (2006.01)    **B01J 8/06** (2006.01)

(52) Classification Coopérative des Brevets (CPC):
**B01J 8/0285; B01J 8/0214; B01J 8/065;
B01J 8/067;** B01J 2208/00159; B01J 2208/00212

(86) Numéro de dépôt international:
**PCT/FR2022/051862**

(87) Numéro de publication internationale:
**WO 2023/057710 (13.04.2023 Gazette 2023/15)**

(54) **RÉACTEUR TUBULAIRE À LIT FIXE COMPORTANT UNE CHAMBRE D'APPOINT**

FESTBETT-ROHRREAKTOR MIT EINER SCHMINKKAMMER

FIXED-BED TUBULAR REACTOR COMPRISING A MAKE-UP CHAMBER

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **05.10.2021 FR 2110518**

(43) Date de publication de la demande:
**14.08.2024 Bulletin 2024/33**

(73) Titulaire: **Commissariat à l'Energie Atomique et
aux Energies
Alternatives
75015 Paris (FR)**

(72) Inventeurs:
• **DUCROS, Frédéric
38054 GRENOBLE CEDEX 09 (FR)**
• **CHAMPON, Isabelle
38054 GRENOBLE CEDEX 09 (FR)**
• **CHAISE, Albin
38054 GRENOBLE Cedex 09 (FR)**
• **BEDEL, Laurent
38054 GRENOBLE CEDEX 09 (FR)**

(74) Mandataire: **Santarelli
Tour Trinity
1 bis Place de la Défense
92400 Courbevoie (FR)**

(56) Documents cités:
**EP-A1- 0 560 157    FR-A1- 3 103 714
US-B2- 8 961 909**

## Description

### DOMAINE TECHNIQUE

La présente invention se rapporte au domaine général des réacteurs échangeurs, et plus particulièrement au domaine des réacteurs échangeurs catalytiques utilisant un catalyseur solide, notamment sous forme de poudre, et destinés à la mise en œuvre de réactions catalytiques endothermiques ou principalement exothermiques.

De telles réactions peuvent notamment être mises en œuvre pour la synthèse de carburants et de combustibles, par exemple des combustibles liquides, tels que le méthanol (MeOH), ou gazeux, tels que le méthane ou substitut de gaz naturel (SNG pour « Synthetic Natural Gas » en anglais), le diméthyléther (DME), les hydrocarbures ou les oléfines, obtenus à partir d'hydrogène et d'oxydes de carbone ou à partir de gaz de synthèse comprenant un mélange d'hydrogène et d'oxydes de carbone. Elles peuvent encore concerner la génération d'hydrogène à partir de méthane, la synthèse d'ammoniac à partir d'hydrogène et d'azote, ou encore les réactions d'hydrogénation et déshydrogénation liées entre l'hydrogène et des molécules de type transporteurs d'hydrogène organique liquide (ou LOHC pour « Liquid Organic Hydrogen Carriers » en anglais).

L'invention peut ainsi être tout particulièrement appliquée à des réactions très exothermiques, telles que celle de méthanation (ou plus généralement la production d'hydrocarbures ou les réactions d'hydrogénation) du monoxyde ou du dioxyde de carbone en présence d'hydrogène. Elle peut également s'appliquer à des réactions du type de celle de Fischer-Tropsch, de celle de reformage humide ou à sec du méthane ou d'autres hydrocarbures, ou encore des réactions d'oxydation ou de déshydrogénation. L'invention peut également être utilisée en tant qu'échangeur de chaleur pour des applications, notamment celles mettant en œuvre un gaz, qui nécessitent des opérations de maintenance fréquentes, par exemple dues à la corrosion, à l'encrassement, entre autres.

L'invention propose ainsi un réacteur tubulaire à lit fixe susceptible de mettre en œuvre notamment des procédés de synthèses organiques exothermiques.

### ÉTAT DE LA TECHNIQUE ANTÉRIEURE

Les réacteurs catalytiques utilisant des catalyseurs solides sont largement mis en œuvre pour la synthèse de composés organiques tels que les carburants de synthèse ou les combustibles parmi lesquels on peut citer les substituts de gaz naturel (SNG), le diméthyléther, le méthanol, les hydrocarbures ou encore les oléfines.

Dans le cadre de la production d'hydrocarbures, ou plus généralement pour les réactions d'hydrogénation, à partir d'hydrogène et d'oxyde de carbone, les équilibres en jeu, ou appelés encore réactions principales par la suite, sont de manière générique les suivantes :

$$Eq.1 : n\,CO + (2n+1)\,H_2 \Leftrightarrow C_nH_{2n+2} + n\,H_2O$$

$$Eq.2 : n\,CO_2 + (3n+1)\,H_2 \Leftrightarrow C_nH_{2n+2} + 2n\,H_2O$$

$$Eq.3 : X + n\,H_2 \Leftrightarrow (-XH_{2n}-)$$

L'équilibre décrit par la troisième équation (Eq.3) concerne les molécules de type transporteurs d'hydrogène organique liquide (ou LOHC pour « Liquid Organic Hydrogen Carriers » en anglais). C'est une réaction réversible qui est donc successivement mise en œuvre dans des séquences exothermiques puis endothermiques. Ces diverses réactions sont largement documentées sur le plan thermochimique. Elles donnent potentiellement lieu à des réactions annexes (par exemple de type WGS (« Water Gas Shift » en anglais ou réaction de changement gaz-eau), RWGS (Reverse Water Gas Shift » en anglais ou réaction inverse de changement gaz-eau), formation de Boudouard, etc.) connues de l'homme du métier et non décrites ici.

Les espèces concernées par ces réactions principales et les réactions annexes sont appelées « réactants » pour les espèces en entrée du réacteur, et « produits » pour les espèces produites par les réactions principales et annexes. De plus, « fluides réactifs » qualifie l'ensemble des espèces concernées par ces réactions. Ces réactions sont toutes associées à des conditions thermodynamiques, pression et température, préférentielles compte-tenu des performances visées : il est généralement intéressant de travailler à pression élevée et il est aussi nécessaire de correctement gérer l'exothermicité de ces réactions en extrayant de la chaleur du domaine réactionnel, ce qui permet de garantir le taux de conversion, la sélectivité, la durée de vie des catalyseurs, entre autres. D'autres fonctionnalités discutées par la suite sont aussi d'intérêt.

On connaît déjà de nombreuses architectures de réacteurs catalytiques utilisés dans l'industrie pour assurer le contrôle et le pilotage thermique de réactions chimiques endothermiques ou exothermiques. Les principaux types de réacteurs échangeurs connus pour les réactions exothermiques sont décrits ci-après.

Tout d'abord, la technologie la plus simple de réacteurs catalytiques est la technologie de réacteurs dits « à lit fixe ». Dans des réacteurs à lit fixe adiabatiques placés en cascade, l'exothermicité est alors généralement gérée par une dilution des réactifs en entrée du premier étage de réaction, par exemple par recirculation des produits, et par la mise en place d'échangeurs de chaleur destinés à refroidir le mélange réactifs-produits entre les différents réacteurs. Cette architecture présente l'avantage de la simplicité de fabrication mais nécessite la mise en place de recycles des gaz pour limiter l'élévation de température et impose l'utilisation de catalyseurs stables à haute température. Ces réacteurs sont plutôt employés comme unités centralisées fonctionnant en régime établi.

Il existe également la technologie des réacteurs échangeurs à lits fluidisés. De tels réacteurs ont été développés pour répondre au problème de transfert thermique dans les lits fixes. Ces réacteurs offrent l'avantage d'une bonne homogénéité thermique dans le réacteur, ce qui évite les points chauds mais nécessitent, à puissance équivalente, un volume de réacteur plus important que dans le cas des lits fixes à écoulement piston. Dans ces réacteurs, le catalyseur est sous forme de particules fines dont l'attrition doit être maitrisée. De plus, la fluidisation des particules impose de limiter les plages de variation de débit de gaz, ce qui rend ces réacteurs peu flexibles vis-à-vis des fonctionnements intermittents.

Une autre technologie de réacteurs échangeurs concerne les réacteurs échangeurs dans lesquels la réaction chimique a lieu au sein d'un canal réactif refroidi continument par un fluide caloporteur extérieur. La plupart de ces réacteurs sont de type tube-calandre, la réaction se produisant dans les tubes réactionnels refroidis en périphérie par un bain caloporteur. Les gaz réactifs circulent axialement dans les tubes qui contiennent un catalyseur, par exemple en poudre.

La combinaison de réacteurs échangeurs du même type ou de types différents au sein d'une même unité peut également être envisagée afin d'améliorer la conversion, la flexibilité ou la valorisation de la chaleur récupérée.

La gestion des contraintes thermiques s'exerçant sur les réacteurs commencent par la réponse au besoin de contrôle thermique. Celle-ci peut emprunter diverses voies comme exposé dans les solutions technologiques précédentes, à savoir des solutions hors réacteur telles que l'étagement de la conversion globale, avec refroidissement et/ou dilution, et/ou condensation intermédiaires ; des solutions dans le réacteur telles que l'évolution vers la notion de réacteurs échangeurs, l'intensification des échanges thermiques, la diminution des tailles des canaux réactifs (millistructuration), l'intégration de structures conductrices 3D pour homogénéisation thermique, l'étagement des injections de réactifs permettant de répartir le dépôt d'énergie.

Quant à la question de la durée de vie des catalyseurs, il faut noter que les réactions exothermiques générant une grande quantité de chaleur, ceci peut conduire à l'apparition de points chauds ayant pour conséquence la dégradation locale du catalyseur et la dégradation des performances de conversion de l'ensemble réacteur/catalyseur. Autrement dit, la dégradation du catalyseur solide peut se traduire par une désactivation de ce dernier et conduire à une réduction du taux de conversion des espèces chimiques en présence. La sélectivité des réactions mises en jeu est également affectée.

Par ailleurs, certaines réactions d'hydrogénation très utiles à l'industrie, comme la synthèse de méthanol ou la synthèse de Fischer-Tropsch, telles que décrites par les équations précédentes Eq.1 à Eq.3 sont équilibrées et ont, pour les conditions thermodynamiques retenues usuellement, des taux de conversion faibles à modérés. Une stratégie intéressante consiste alors à associer des membranes permsélectives à une ou des parois des canaux réactifs, de manière à soustraire une espèce produite, typiquement l'eau, au milieu réactif, ce qui déséquilibre la réaction et augment sa productivité. L'avantage d'un tel procédé a pu être démontré expérimentalement et théoriquement. Les innovations peuvent porter sur deux éléments principaux : les membranes et les méthodes d'intégration aux réacteurs.

Dans la solution classique de réacteur échangeur avec le catalyseur localisé dans les tubes, pour des technologies de type tube-calandre, un des problèmes est le contrôle des zones rendues plus chaudes à cause de l'exothermie de la réaction. Ce phénomène nécessite de refroidir intensément les tubes sur toutes leur surface alors qu'à un instant donné, seule une faible partie de cette surface nécessite d'être refroidie intensément, par exemple pour une injection et une circulation axiale des réactifs. Une conséquence de cela est un surdimensionnement du débit de fluide thermique.

Afin de pallier ces problèmes, il a été proposé un agencement permettant de répartir la distribution des réactifs sur toute la longueur des tubes. Cette solution permet alors d'obtenir une meilleure homogénéité de la température sur toute la longueur du réacteur. A cet égard, les documents US 3,758,279 A, US 4,374,094 A, EP 0 560 157 A1 et US 2,997,374 A proposent des réacteurs échangeurs mettant en œuvre une distribution des réactifs à partir d'un espace de distribution annulaire. Notamment, ces réacteurs échangeurs, de forme généralement cylindrique, comprennent, agencés de manière coaxiale et à partir de l'extérieur du réacteur, un tube, l'espace de distribution annulaire, une charge de catalyseur et un espace de collecte. Ces solutions constituent le standard du refroidissement dans des réacteurs de type tube et calandre, c'est-à-dire que le refroidissement des tubes est piloté uniquement par l'écoulement dans la calandre.

Ces agencements ne sont toutefois pas satisfaisants. En effet, la présence de l'espace de distribution annulaire disposé autour de la charge de catalyseur limite les transferts de chaleur du catalyseur vers le tube, rendant peu efficace les systèmes de refroidissement généralement considérés. Il reste néanmoins possible d'insérer des éléments conducteurs de chaleur dans le réacteur. Une telle solution reste toutefois incompatible avec les réacteurs comprenant des tubes de faible diamètre.

Inversement, le document CN 103990420 A propose de mettre en œuvre un insert pourvu d'une chambre de distribution et d'une chambre de collecte, disposée au centre d'un tube et définissant avec ce dernier une espace annulaire logeant le catalyseur solide. Toutefois, l'agencement proposé dans ce document ne permet pas de répartition homogène de la température au sein de l'espace annulaire. Plus particulièrement, cet agencement ne permet pas d'obtenir un profil de température optimal au sein du catalyseur solide.

La figure 1 du document US 8,961,909 A représente un autre exemple de réacteur du type tube-calandre. Ce réacteur est notamment pourvu d'un tube d'injection, plongé dans un lit de poudre catalytique, et le long duquel sont ménagés des perçages. Ces derniers sont notamment agencés pour assurer une injection du gaz réactif à différents niveaux du lit de poudre catalytique, et ainsi limiter l'apparition de points chauds dans ledit lit. Toutefois, ce réacteur n'est pas satisfaisant. En effet, afin d'assurer son refroidissement, ce réacteur requiert la mise en place d'une pluralité de circuits de circulation d'un fluide caloporteur, ce qui augmente d'autant sa complexité.

Le document US 7,402,719 B2, notamment à la figure 3a, divulgue un autre exemple de réacteur agencé pour permettre une injection étagée d'un réactif C en vue de sa réaction avec un réactif A. Ce réacteur comprend à cet égard deux nappes (ou canaux) séparées par une paroi et destinées à assurer la circulation, respectivement, du réactif A et du réactif C. Les deux nappes sont par ailleurs en communication fluidique au moyen d'une pluralité de perçages ménagés dans la paroi les séparant. Ces perçages sont notamment agencés afin d'assurer un mélange progressif du réactif C avec le réactif A. Ce mélange progressif permet ainsi de limiter l'apparition de points chauds. Cependant, l'agencement du réacteur sous forme d'un empilement de nappes rend ce dernier peu compact.

En outre, dans la demande de brevet FR 3 103 714 A1 de la Demanderesse, un dispositif de type insert organise une distribution axiale des gaz réactifs dans les tubes, une circulation des gaz dans la direction sensiblement orthoradiale à travers un lit de catalyseur dans un espace annulaire défini par la paroi interne d'un tube et la paroi externe de l'insert, et une collecte axiale des gaz produits.

Il existe encore un besoin pour améliorer ces types de réacteurs, et notamment pour doter de tels réacteurs tubes/calandre d'une circulation de fluide alternative permettant d'améliorer notamment le refroidissement.

## EXPOSÉ DE L'INVENTION

L'invention a donc pour but de remédier au moins partiellement aux besoins mentionnés précédemment et aux inconvénients relatifs aux réalisations de l'art antérieur.

L'invention vise notamment à proposer un réacteur tubulaire à lit fixe permettant une distribution plus uniforme des réactifs au sein du catalyseur solide, une répartition plus homogène du flux de chaleur généré au sein du catalyseur solide, une meilleure gestion du refroidissement. De plus, l'invention cherche à proposer un réacteur tubulaire pour lequel la fiabilité et la durée de vie (des catalyseurs) sont améliorées au regard des réacteurs connus de l'état de la technique, et permettant d'optimiser (augmenter) le temps de passage des gaz dans le lit fixe de poudre catalytique.

L'invention a ainsi pour objet, selon l'un de ses aspects, un réacteur tubulaire à lit fixe qui s'étend, selon un axe longitudinal, entre une première extrémité et une deuxième extrémité, ledit réacteur comprenant un lit de poudre catalytique confiné dans un espace annulaire délimité par une paroi externe d'un tube creux et une paroi interne d'un insert creux disposé de manière coaxiale dans le tube creux, l'insert creux comprenant au moins une chambre de distribution et au moins une chambre de collecte, séparées l'une de l'autre par au moins une première paroi séparatrice, ladite au moins une chambre de distribution et ladite au moins une chambre de collecte comprenant, respectivement, une ouverture d'admission de gaz au niveau de la première extrémité et une ouverture d'évacuation de gaz au niveau de la deuxième extrémité,
caractérisé en ce que le réacteur comporte en plus au moins une chambre d'appoint séparée de ladite au moins une chambre de distribution et de ladite au moins une chambre de collecte par au moins une première paroi séparatrice, ladite au moins une chambre d'appoint comprenant un orifice d'entrée d'au moins un fluide d'appoint, distinct des gaz circulant dans ladite au moins une chambre de distribution et dans ladite au moins une chambre de collecte, au niveau de la première extrémité et un orifice de sortie dudit au moins un fluide d'appoint au niveau de la deuxième extrémité.

Grâce à l'invention, il est notamment possible d'améliorer le refroidissement de la zone réactionnelle (réactifs, produits de réaction et catalyseurs) en permettant de refroidir cette zone par une circulation interne et externe de fluide caloporteur, ce qui est bénéfique pour l'ensemble des réactions d'hydrogénation par exemple. Les performances globales du réacteur (taux de conversion et sélectivité des réactions) seront améliorées par une meilleure homogénéité de la température résultant de l'application de l'invention.

Le réacteur selon l'invention peuvent en outre comporter l'une ou plusieurs des caractéristiques suivantes prises isolément ou suivant toutes combinaisons techniques possibles.

Chacune desdites au moins une chambre de distribution, au moins une chambre de collecte et au moins une chambre d'appoint peut être délimitée par une section de la paroi interne et deux premières parois séparatrices.

De plus, le réacteur peut comporter au niveau de la première extrémité et au niveau de la deuxième extrémité, respectivement, un espace distributeur et un espace collecteur entre lesquels l'insert creux est disposé.

En outre, le tube et l'insert peuvent être maintenus par au moins deux plaques tubulaires de maintien respectivement au niveau des première et deuxième extrémités, au moins l'une desdites au moins deux plaques tubulaires de maintien, notamment toutes les plaques tubulaires de maintien, comportant un conduit d'entrée ou de sortie respectivement relié fluidiquement à un orifice d'entrée ou de sortie de fluide d'appoint et formé dans ladite au moins l'une desdites au moins deux plaques tubulaires de maintien, pour permettre notamment une admission et/ou une extraction latérale de fluide d'appoint.

Les longueurs axiales du tube et de l'insert peuvent être différentes, la longueur axiale du tube étant notamment plus courte que la longueur axiale de l'insert.

En variante, le tube et l'insert peuvent être maintenus par au moins deux plaques tubulaires de maintien respectivement au niveau des première et deuxième extrémités, et au moins un conduit d'entrée ou de sortie respectivement relié fluidiquement à un orifice d'entrée ou de sortie de fluide d'appoint peut être situé respectivement dans l'espace distributeur et dans l'espace collecteur, hors desdites au moins deux plaques tubulaires de maintien, pour permettre notamment une admission et/ou une extraction latérale de fluide d'appoint.

En variante encore, le tube et l'insert peuvent être maintenus par au moins deux plaques tubulaires de maintien respectivement au niveau des première et deuxième extrémités, et ledit orifice d'entrée et ledit orifice de sortie dudit au moins un fluide d'appoint peuvent être respectivement formés dans les extrémités supérieure et inférieure de l'insert pour permettre respectivement une alimentation depuis l'espace distributeur et une extraction depuis l'espace collecteur, le réacteur comprenant en outre au moins un conduit d'alimentation latéral de gaz et au moins un conduit d'extraction latéral de gaz.

Ledit au moins un conduit d'alimentation latéral et l'alimentation en fluide d'appoint de l'espace distributeur d'une part, et ledit au moins un conduit d'extraction latéral et l'extraction en fluide d'appoint de l'espace collecteur d'autre part, peuvent être séparés par une plaque de séparation étanche.

En outre, un joint d'étanchéité peut être disposé entre l'insert et chacune des plaques de séparation étanches.

Par ailleurs, l'écoulement dudit au moins un fluide d'appoint peut être réalisé de manière co-courante ou contre-courante à l'écoulement des gaz dans lesdites au moins une chambre de distribution et de collecte.

Le cas échéant, la poudre catalytique peut être retenue dans l'espace annulaire par un joint en matière fibreuse au niveau de chacune des extrémités de l'espace annulaire.

Avantageusement, ledit au moins un fluide d'appoint peut être un fluide caloporteur, notamment un fluide de refroidissement.

De plus, l'insert peut être une pièce monobloc.

Par ailleurs, l'invention a encore pour objet, selon un autre de ses objets, l'utilisation d'un réacteur tubulaire tel que défini précédemment, caractérisée en ce qu'elle met en œuvre des réactions endothermiques ou exothermiques de synthèse de carburants et de combustibles.

En particulier, l'espace annulaire peut permettre une réaction d'hydrogénation et ladite au moins une chambre d'appoint peut permettre une réaction de déshydrogénation, ou l'inverse.

## BRÈVE DESCRIPTION DES DESSINS

L'invention pourra être mieux comprise à la lecture de la description détaillée qui va suivre, d'exemples de mise en œuvre non limitatifs de celle-ci, ainsi qu'à l'examen des figures, schématiques et partielles, du dessin annexé, sur lequel :

- la figure 1 est une représentation schématique d'un réacteur tubulaire à lit fixe conforme à l'invention, selon un plan en coupe, passant par l'axe longitudinal du réacteur, en particulier selon le plan de coupe longitudinale PP de la figure 2, permettant de visualiser une chambre de collecte et une chambre de distribution,
- la figure 2 est une vue en coupe selon un plan transversal, ou section normale, perpendiculaire à l'axe longitudinal du réacteur tubulaire de la figure 1,
- la figure 3 est une représentation schématique du réacteur tubulaire des figures 1 et 2 selon le plan de coupe longitudinale P'P' de la figure 2, permettant de visualiser deux chambres d'appoint, alimentées par des orifices d'entrée et de sortie latéraux,
- la figure 4 est une vue semblable à celle de la figure 1 permettant d'illustrer l'utilisation de joints pour retenir le catalyseur,
- la figure 5 est une vue semblable à celle de la figure 3 permettant d'illustrer l'utilisation de joints pour retenir le catalyseur,
- la figure 6 est une représentation schématique d'un autre exemple de réacteur tubulaire conforme à l'invention, selon une vue semblable à celle de la figure 1,
- la figure 7 est une vue semblable à celle de la figure 3 pour le réacteur de la figure 6,
- la figure 8 est encore une représentation schématique d'un autre exemple de réacteur tubulaire conforme à l'invention, selon une vue semblable à celle de la figure 1,
- la figure 9 est une vue semblable à celle de la figure 3 pour le réacteur de la figure 8,
- les figures 10 et 11 sont des vues, en coupe axiale respectivement selon les plans PP et P'P' en référence à la figure 2, d'une pluralité de réacteurs tubulaires semblables à celui des figures 1 à 5, situés à l'intérieur d'une calandre, et
- la figure 12 est une vue, en coupe axiale selon le plan P'P' en référence à la figure 2, d'une pluralité de réacteurs tubulaires semblables à celui des figures 6 et 7, situés à l'intérieur d'une calandre.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

## EXPOSÉ DÉTAILLÉ DE MODES DE RÉALISATION PARTICULIERS

La présente invention concerne un réacteur échangeur tubulaire à lit de poudre catalytique fixe. En particulier, le lit de poudre catalytique est confiné dans un espace annulaire délimité par une paroi, dite paroi externe, d'un tube creux et une autre paroi, dite paroi interne, d'un insert creux logé de manière coaxiale dans ledit tube. Le lit de poudre catalytique peut notamment comporter un catalyseur sous forme de grains.

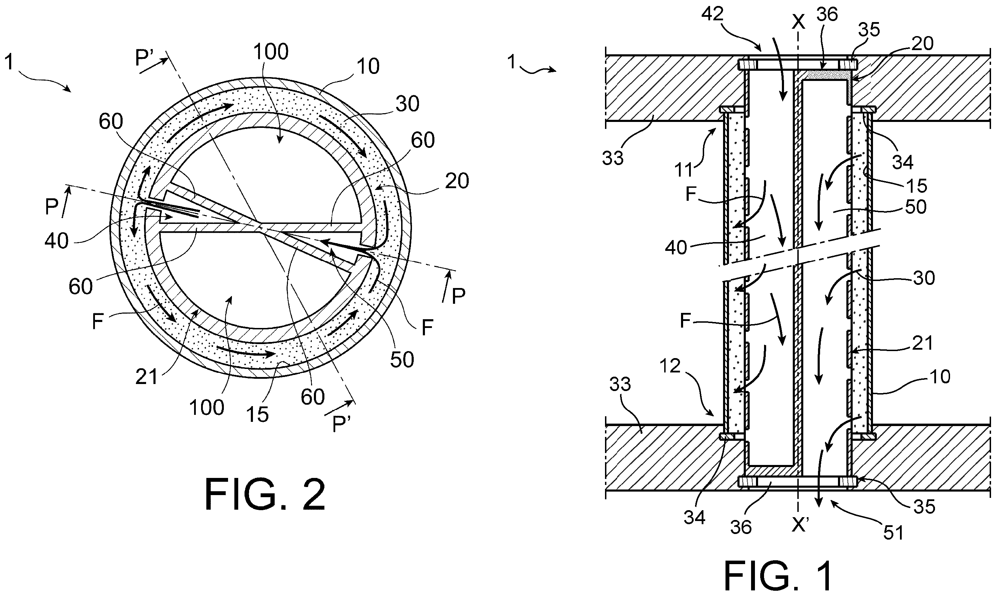

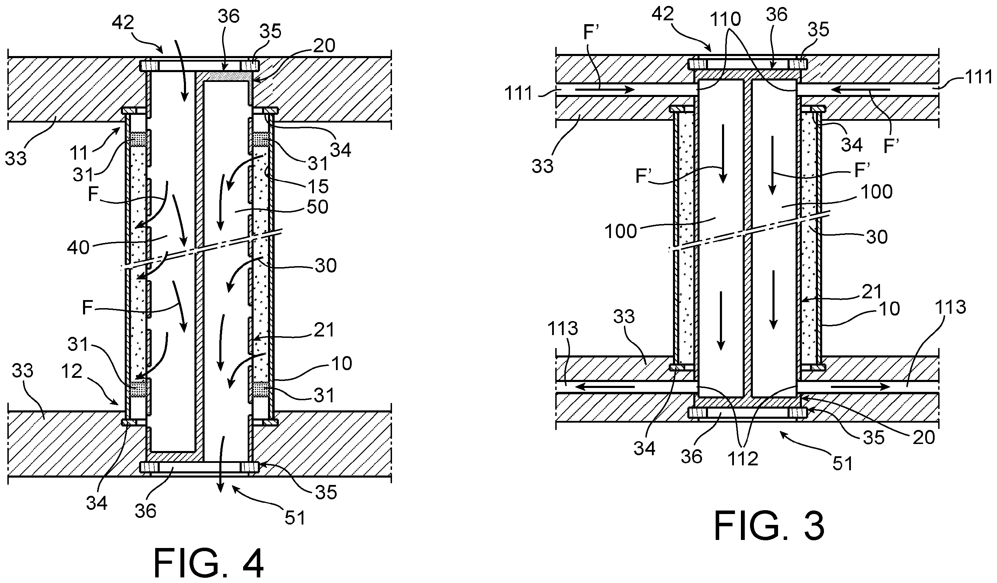

Ainsi, aux figures 1 et 2, on peut voir un exemple de réalisation d'un réacteur tubulaire à lit fixe selon la présente invention. Il est à noter que sur ces figures 1 et 2, ainsi que sur l'ensemble des figures décrites par la suite, les flèches F représentent le sens de parcours du gaz. Par ailleurs, les flèches F', notamment visibles sur les figures 3, 5, 7, 9 et 11, représentent le sens de parcours du fluide d'appoint.

Le réacteur tubulaire 1 selon la présente invention comprend un tube creux 10 externe qui s'étend selon un axe longitudinal XX' entre une première extrémité 11 et une deuxième extrémité 12. Le tube creux 10 peut présenter une symétrie de révolution autour de l'axe longitudinal XX'. Il est donc entendu que l'axe longitudinal XX' peut être un axe de révolution du tube creux 10.

Le tube creux 10 peut comprendre un métal, et notamment un métal choisi parmi : acier, alliage d'aluminium, de cuivre, de nickel, entre autres. Le diamètre du tube creux 10 peut être compris entre 5 mm et 100 mm. La paroi, dite paroi externe 15, formant le tube creux 10 peut présenter une épaisseur comprise entre 0,5 mm et 10 mm. Le tube creux 10 peut présenter une longueur comprise entre 10 fois et 200 fois le diamètre de la surface interne.

Le réacteur tubulaire 1 comprend également un insert creux 20 qui s'étend également selon l'axe longitudinal XX' et présente une forme généralement cylindrique. L'insert creux 20 est notamment logé dans le volume du tube creux 10 de manière coaxiale à ce dernier. En particulier, l'insert 20 comprend également une paroi, dite paroi interne 21, en particulier une paroi éventuellement perméable au gaz, qui délimite avec la paroi externe 15 un espace annulaire 30. L'espace annulaire 30 est, à cet égard, rempli d'une poudre catalytique, et sera le siège des réactions de conversion de gaz réactifs susceptibles de transiter dans le réacteur tubulaire 1. L'espace annulaire 30 peut présenter une épaisseur, définie comme la distance entre la paroi externe 15 et la paroi interne 21, comprise entre 2% et 20 % du diamètre de la surface interne du tube creux 10. L'insert creux 20 peut être une pièce monobloc. L'insert creux 20 peut par exemple être réalisé en acier inoxydable, notamment par des méthodes de brasage, ou en aluminium, notamment par des méthodes d'extrusion ou de fabrication additive (par exemple un procédé de fabrication 3D), voire en polymères pour certaines réactions à faible température. Les diverses ouvertures de l'insert creux 20 peuvent être réalisées lors de la fabrication de l'insert, et/ou réalisées par usinage dans un second temps.

L'insert creux 20 comprend par ailleurs au moins une chambre de distribution 40 et au moins une chambre de collecte 50. Ici, par souci de simplicité, une seule chambre de distribution 40 avec un seul point d'injection et une seule chambre de collecte 50 sont représentées mais ce choix n'est pas limitatif. En particulier, l'insert creux 20 peut comprendre notamment entre 1 et 4 chambres de distribution 40, et entre 1 et 4 chambres de collecte 50.

Ladite au moins une chambre de distribution 40 et ladite moins une chambre de collecte 50 sont avantageusement disposées en alternance, et s'étendent sur toute la longueur de l'insert creux 20.

Avantageusement, l'insert creux 20 comprend encore au moins une chambre d'appoint 100 organisant la circulation axiale d'un fluide d'appoint, ici deux chambres d'appoint 100, mais l'invention n'est pas limitée sur le nombre de chambres et de fluides d'appoint supplémentaires.

Ainsi, on trouve successivement par observation d'un plan en section normale à l'axe XX' du tube creux 10, comme visible sur la figure 2, une première chambre d'appoint 100, ladite au moins une chambre de collecte 50, une deuxième chambre d'appoint 100 et ladite au moins une chambre de distribution 40. Ladite au moins une chambre de collecte 50, ladite au moins une chambre de distribution 40 et les chambres d'appoint 100 sont par ailleurs séparées les unes des autres par des premières parois séparatrices 60. Il est donc entendu qu'une chambre de distribution 40 est délimitée par deux parois séparatrices 60 et une section de la paroi interne 21. De manière équivalente, une chambre de collecte 50 est également délimitée par deux premières parois séparatrices 60 et une autre section de la paroi interne 21. De manière encore équivalente, une chambre d'appoint 100 est séparée par deux premières parois séparatrices 60 et encore une autre section de la paroi interne 21.

En outre, les premières parois séparatrices 60 s'étendent sur toute la longueur de l'insert creux 20 dans le volume défini par le tube creux 10, et sont agencées pour prévenir tout passage direct de gaz d'une chambre à l'autre. Par exemple, les premières parois séparatrices 60 forment des plans passant par l'axe longitudinal XX'.

Notamment, les deux premières parois séparatrices 60 d'une chambre de distribution 40 peuvent présenter une forme généralement allongée et s'étendre selon l'axe longitudinal XX' de la première extrémité 11 vers la deuxième extrémité 12. En particulier, les deux premières parois séparatrices 60 d'une chambre de distribution 40 peuvent présenter un côté commun en coïncidence avec l'axe longitudinal XX'.

En outre, le réacteur 1 peut comporter, au niveau de la première extrémité 11 de l'insert creux 20, un espace distributeur 42, ou plenum d'entrée, par lequel un ou des gaz réactifs sont susceptibles d'être admis dans la chambre de distribution 40 au travers d'une ouverture d'admission. De même, le réacteur 1 peut comporter, au niveau de la deuxième extrémité 12 de l'insert creux 20, un espace collecteur 51, ou plenum de sortie, par lequel un ou plusieurs gaz sont susceptibles d'être évacués au travers d'une ouverture d'évacuation.

Par ailleurs, la chambre de distribution 40 est obturée au niveau de la deuxième extrémité 12, et la chambre de collecte 50 est obturée au niveau de la première extrémité 11.

La paroi interne 21 peut en outre comprendre au moins une ouverture distributrice et au moins une ouverture collectrice, permettant respectivement la distribution d'un gaz susceptible d'être admis par l'ouverture d'admission au niveau du plenum d'entrée 42 dans un compartiment de distribution vers l'espace annulaire 30, et la collecte du gaz distribué dans l'espace annulaire 30 par la chambre de collecte 50.

Le tube creux 10 est avantageusement maintenu par deux plaques tubulaires de maintien 33 lesquelles comportent chacune un premier système de fixation étanche 34 du tube creux 10 à chaque plaque de maintien 33. De même, l'insert creux 20 est avantageusement maintenu par les deux plaques de maintien 33 lesquelles comportent chacune un deuxième système de fixation étanché 35 de l'insert creux 20 à chaque plaque de maintient 33. Des parois de séparation 36 sont également présentes dans chacune des plaques de maintien 33, entre lesquelles sont contenus le tube creux 10 et l'insert creux 20.

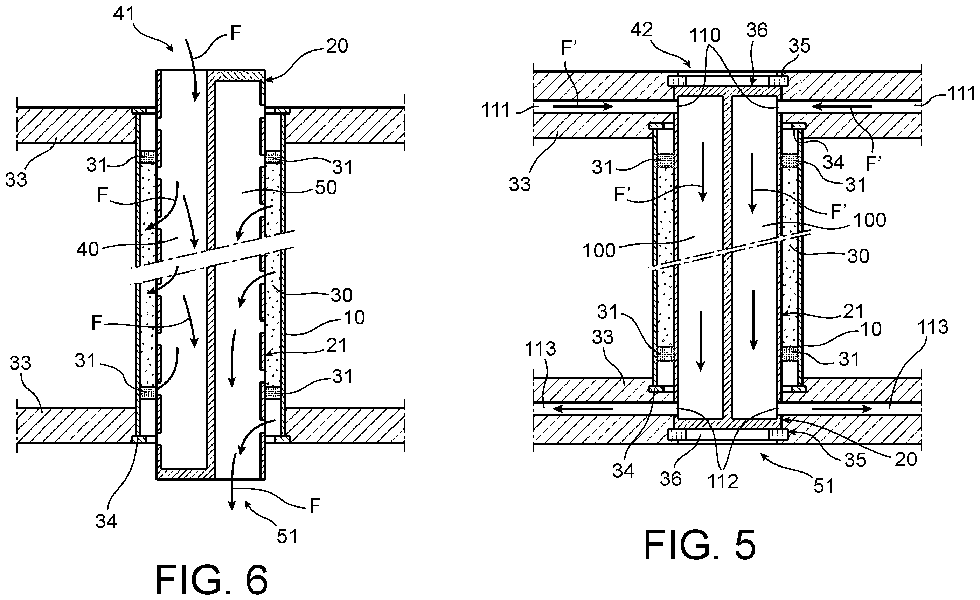

Selon un aspect avantageux illustré aux figures 4 et 5, la poudre catalytique est retenue dans l'espace annulaire 30 par un joint 31, par exemple en matière fibreuse, au niveau de chacune des extrémités de l'espace annulaire 30. Dans la mesure où le joint 31 est en matière fibreuse, ce dernier est nécessairement poreux et donc perméable aux gaz réactifs. La matière fibreuse peut à cet égard comprendre au moins un des éléments choisi parmi : fibre de verre, fibre de céramique, fibre de métal, fibre de carbone, fibre de matériau polymère.

Le joint 31 peut notamment être sous forme d'une tresse, d'une gaine, d'une cordelette ou simplement comprendre un bourrage de la matière fibreuse. La matière fibreuse est avantageusement un isolant thermique et présente une conductivité thermique sensiblement équivalente à celle du catalyseur utilisé (0,2 W/m/K à 10 W/m/K).

La figure 3 permet de visualiser, en coupe axiale selon le plan P'P' de la figure 2, les chambres d'appoint 100 décrites précédemment. Chaque chambre d'appoint 100 est alimentée par un orifice d'entrée latéral 110, situé à proximité de l'extrémité 11 du tube creux 10, cet orifice d'entrée 110 étant alimenté par un conduit d'entrée latéral 111 formé dans la plaque de maintien 33 supérieure.

De même, chaque chambre d'appoint 100 comporte un orifice de sortie latéral 112, situé à proximité de l'extrémité 12 du tube creux 10, cet orifice de sortie 112 étant relié fluidiquement à un conduit de sortie latéral 113 formé dans la plaque de maintien 33 inférieure. De cette manière, un fluide d'appoint F' peut être admis par circulation dans les plaques tubulaires de maintien 33.

De façon avantageuse, l'invention permet ainsi d'étendre les capacités de thermalisation du réacteur 1, en particulier de permettre de gérer et de faire circuler des fluides d'appoint, notamment des fluides utilitaires ou réactifs, en plus des réactifs et produits de réaction, par exemple selon les équations Eq. 1 et/ou Eq. 2 décrites précédemment.

La géométrie de l'insert creux 20 et la géométrie du tube externe creux 10 sont définies de manière à permettre une alimentation séparée des chambres de distribution 40 et d'appoint 100, et des sorties séparées des chambres de collecte 50 et d'appoint 100.

Dans l'exemple de réalisation des figures 1 à 5, le tube creux 10 et l'insert creux 20 ont des longueurs axiales différentes, le tube creux 10 étant moins long que l'insert creux 20, et l'alimentation et l'évacuation en fluide d'appoint se fait au travers d'une plaque de maintien 33, par le biais des conduits 111 et 113. De plus, l'écoulement du fluide d'appoint F' est dirigé axialement et de manière co-courante de la circulation des gaz. Toutefois, un concept à contre-courant peut également être intéressant.

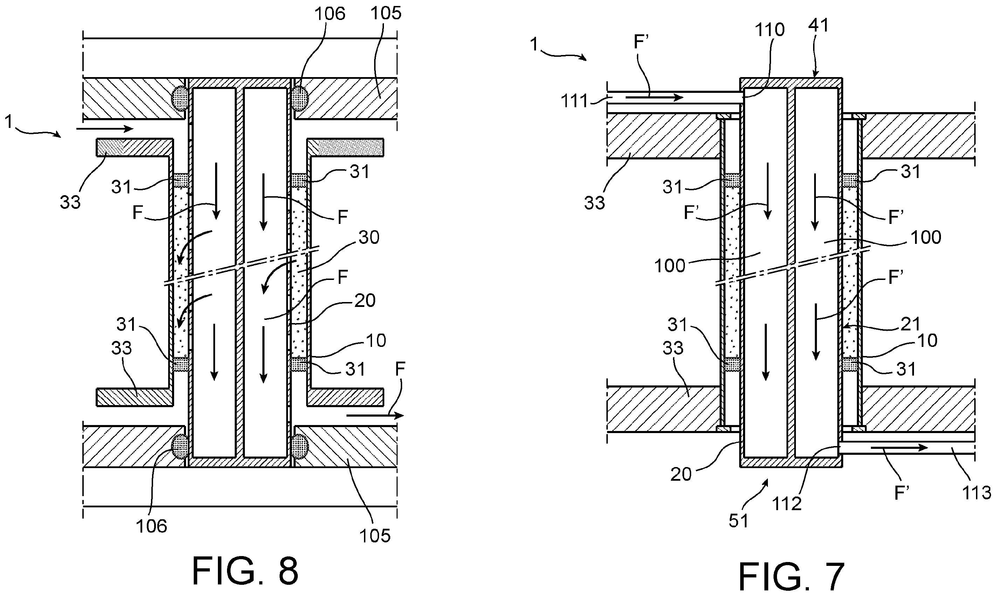

Dans l'exemple de réalisation des figures 6 et 7, l'alimentation des chambres d'appoint 100 est assurée par le biais de conduits dédiés situés dans les plenums d'entrée 42 et de sortie 51, et non plus par le biais de conduits usinés dans des plaques de maintien 33.

Plus précisément, comme visible sur la figure 7, un conduit d'entrée latéral 111, pour l'admission de fluide d'appoint F', est situé le long de la plaque de maintien 33 supérieure, hors de celle-ci, dans la partie haute du réacteur 1. De même, un conduit de sortie latéral 112, pour l'extraction de fluide d'appoint F', est situé le long de la plaque de maintien 33 inférieure, hors de celle-ci, dans la partie basse du réacteur 1.

Ainsi, les alimentations et évacuations du fluide d'appoint F' sont assurées par des conduits 111, 112 réalisés hors des plaques tubulaires de maintien 33, et assurant une liaison étanche avec l'extérieur des plenums d'entrée 41 et de sortie 51. De plus, l'écoulement du fluide d'appoint F' est dirigé axialement et de manière co-courante de la circulation des gaz. Toutefois, un concept à contre-courant peut également être intéressant.

Dans l'exemple de réalisation des figures 7 et 8, l'alimentation des chambres d'appoint 100 est assurée par le biais de piquages supplémentaires dans les plenums d'entrée 42 et de sortie 51.

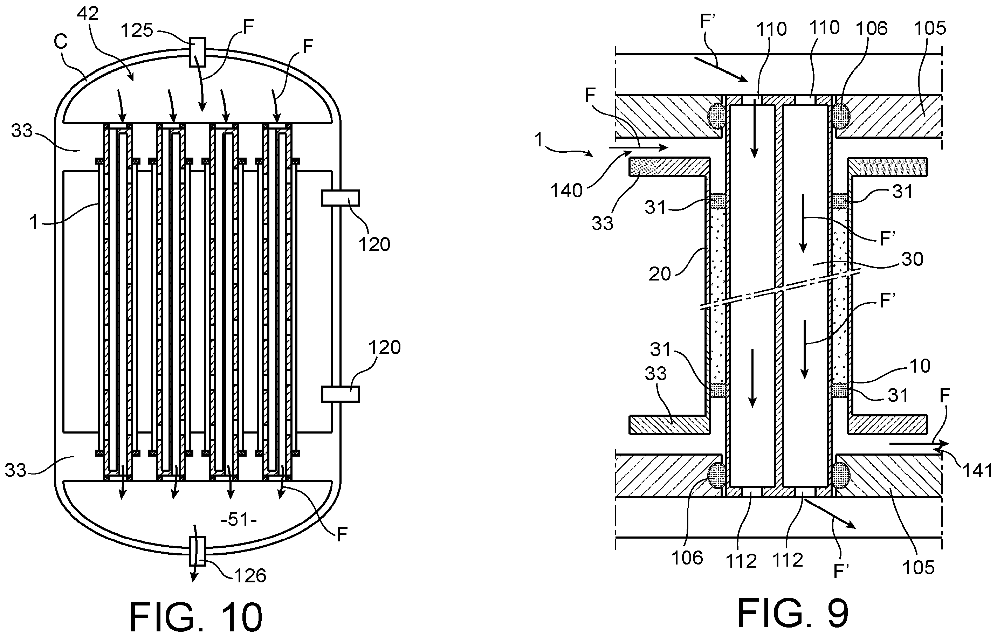

Plus précisément, comme visible sur la figure 7, l'alimentation et l'extraction en fluide d'appoint F' sont assurées par les extrémités supérieure et inférieure de l'insert creux 20. Les fluides réactifs F sont alors introduits latéralement par le biais de conduits latéraux d'admission 140 et d'extraction 141 de fluides réactifs, comme visible sur la figure 9. La séparation étanche entre les fluides d'appoint F' et les fluides réactifs F est assurée par le biais de plaques de séparation 105, démontables, introduites dans les plenums d'entrée et de sortie, et disposées chacune autour de l'insert creux 20. Un joint d'étanchéité 106 est alors placé entre chaque plaque de séparation 105 et l'insert creux 20.

De façon avantageuse, les modes de réalisation des figures 5 et 6 d'une part, et des figures 7 et 8 d'autre part, permettent de réaliser un assemblage plaque tubulaire et tubes proche des réalisations classiques.

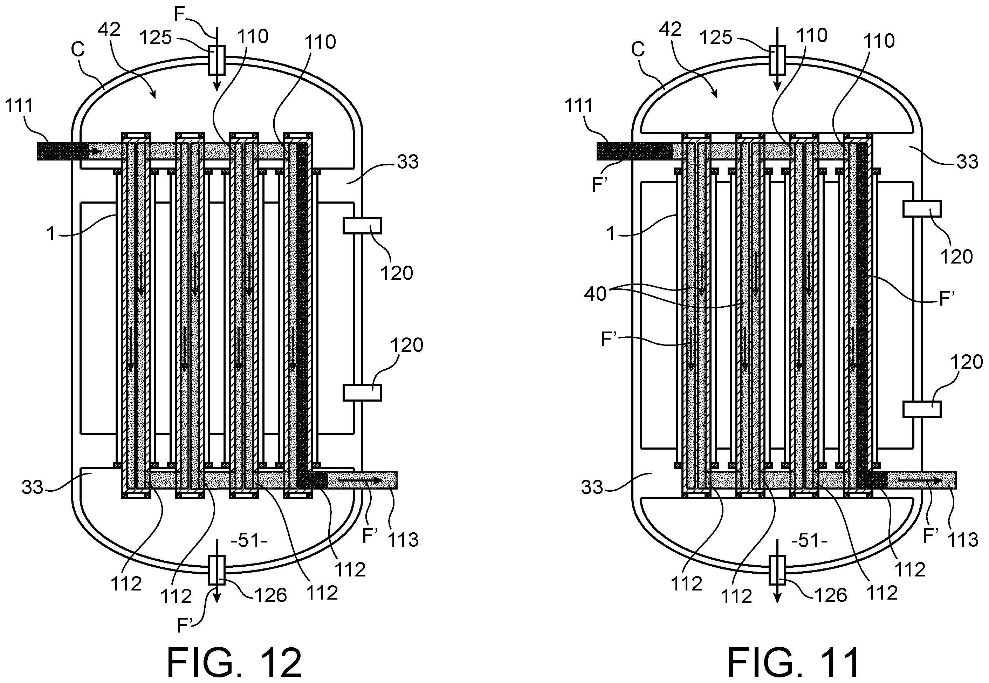

Les figures 10 et 11 sont une illustration de la mise en œuvre d'une pluralité de réacteurs tubulaires 1 conformes à l'invention, et notamment selon la variante des figures 1 à 5. De même, la figure 12 est une illustration de la mise en œuvre d'une pluralité de réacteurs tubulaires 1 conformes à l'invention selon la variante des figures 6 et 7.

Cette mise en œuvre comprend notamment quatre tubes 1 disposés parallèlement les uns aux autres dans une calandre C. Les plaques de maintien tubulaires 33 permettent de maintenir les tubes 1, et de ménager un espace de circulation d'un fluide caloporteur destiné au refroidissement des tubes 1, par le biais de systèmes d'alimentation et d'évacuation de fluide caloporteur 120.

Dans l'exemple des figures 10 et 11, la ou les chambres de distribution 40 sont alimentées directement depuis le plenum d'entrée 42 par le biais de systèmes d'alimentation en réactifs 125. Puis, l'évacuation des produits et réactifs non convertis est réalisée dans le plenum de sortie 51 par le biais de systèmes d'extraction 126.

Ici, les tubes externes sont plus courts que les inserts 20, et le fluide d'appoint F' est distribué directement dans les inserts 20 par des conduits 111, 113 intégrés aux plaques de maintien tubulaires 33.

Dans l'exemple de la figure 12, les inserts creux 20 sont plus longs. L'alimentation et l'extraction du fluide d'appoint F' sont réalisées par un circuit dédié de conduits 111, 113 situés hors des plaques de maintien 33.

Le réacteur tubulaire 1 selon la présente invention, et notamment la mise en œuvre de chambres d'appoint 100 permettant la circulation de fluides utilitaires ou réactifs, permet d'étendre les capacités de thermalisation.

Cette configuration répond de plus favorablement à la problématique d'échauffement de la poudre catalytique, notamment sous forme de catalyseur en grains, et limite ainsi l'apparition de points chauds. Il en résulte un dispositif plus performant et plus durable. En outre, la disposition de la poudre catalytique dans l'espace annulaire 30 facilite le refroidissement de cette dernière.

Exemple d'application :

Deux modélisations numériques ont été réalisées sur le principe de l'invention avec comme fluide d'appoint un fluide caloporteur de refroidissement.

Pour celles-ci, il a été supposé une alimentation en réactants à 3 bar et 250°C, un coefficient d'échange de chaleur homogène en extérieur du tube creux 10 de 1500 $W/m^2/K$ avec une température de référence de 250°C, et un dégagement de chaleur dans la matrice poreuse de 200 W de puissance.

Une première simulation a été conduite sans circulation interne de caloporteur, et une seconde simulation avec circulation interne de caloporteur, toutes choses étant égales par ailleurs. Les résultats sont résumés dans le Tableau 1 ci-dessous. Non seulement la circulation interne d'un fluide d'appoint, ici un fluide caloporteur, dans les chambres d'appoint 100 permet de limiter l'augmentation des températures mais elle permet aussi une meilleure homogénéité des températures dans le lit catalytique.

*Tableau 1*

| fluide caloporteur (d'appoint) | gaz | | matrice poreuse (catalyseur) | |
|---|---|---|---|---|
| | température moyenne (°C) | température maximale (°C) | température moyenne (°C) | température maximale (°C) |
| Sans | 271 | 292 | 290 | 308 |
| Avec | 254 | 256 | 271 | 279 |

Bien entendu, l'invention n'est pas limitée aux exemples de réalisation qui viennent d'être décrits. Diverses modifications peuvent y être apportées par l'homme du métier.

En particulier, le nombre, et la disposition respective et angulaire, des chambres d'appoint 100, de collecte 50 et de distribution 40 peut varier.

Le sens de circulation des fluides d'appoint F' dans les chambres d'appoint 100 peut varier.

Le choix de la localisation des alimentations et extractions, notamment en extrémité d'insert 20 ou par piquage latéral de l'insert 20, respectivement des fluides d'appoint F' et de réactifs F peut varier en fonction du procédé.

L'alimentation des chambres d'appoint 100 peut ou non être associée aux plaques tubulaires 33.

Le réacteur 1 selon l'invention peut ou non comprendre des moyens d'injection étagée.

Pour le cas où les chambres d'appoint 100 sont utilisées comme chambres de refroidissement, comme dans les exemples précédents, l'efficacité du système peut encore être amélioré par diverses manipulations visant à intensifier les échanges thermiques et connues par l'homme du métier, telles que structuration, notamment micro-structuration, de surfaces, modifications des régimes thermo-hydrauliques, entre autres.

Pour le cas où l'insert creux 20 serait le siège d'une réaction endothermique, l'apport de chaleur pourrait être réalisé par le fluide caloporteur, en lieu et place du fluide d'appoint en tant que fluide de refroidissement.

En particulier, les chambres d'appoint 100 peuvent être utilisées pour y abriter une réaction chimique de nature inverse à celle localisée dans l'espace annulaire 30, par exemple une réaction d'hydrogénation contre une réaction de déshydrogénation, dans le but d'une intégration énergétique. Cette dernière réaction peut par exemple être envisagée comme se déroulant au travers d'un lit fixe.

Ainsi, à titre d'exemple, l'espace annulaire 30 peut être le siège d'une réaction d'hydrogénation de type $CO_2 + 4 H_2 \rightarrow CH_4 + 2 H_2O$ ($\Delta H_{298}$ = -165 kJ/mol), et les chambres d'appoint 100 peuvent être le siège d'une réaction de déshydrogénation sur lit fixe catalytique, basée sur le couple GBL/BDO (« butyrolactone / butanediol ») de molécules de type transporteurs d'hydrogène organique liquide (LOHC) de type $BDO \rightarrow GBL + 2 H_2$ ($\Delta H_{298}$ = 31 kJ/mol de $H_2$). Les enthalpies de réaction, et les conditions opératoires, sont compatibles pour que l'énergie nécessaire à la déshydrogénation soit fournie par la réaction d'hydrogénation.

**Revendications**

1. Réacteur tubulaire (1) à lit fixe qui s'étend, selon un axe longitudinal (XX'), entre une première extrémité (11) et une deuxième extrémité (12), ledit réacteur (1) comprenant un lit de poudre catalytique confiné dans un espace annulaire (30) délimité par une paroi externe (15) d'un tube creux (10) et une paroi interne (21) d'un insert creux (20) disposé de manière coaxiale dans le tube creux (10), l'insert creux (20) comprenant au moins une chambre de distribution (40) et au moins une chambre de collecte (50), séparées l'une de l'autre par au moins une première paroi séparatrice (60), ladite au moins une chambre de distribution (40) et ladite au moins une chambre de collecte (50) comprenant, respectivement, une ouverture d'admission (42) de gaz au niveau de la première extrémité (11) et une ouverture d'évacuation (51) de gaz au niveau de la deuxième extrémité (12),
**caractérisé en ce que** le réacteur (1) comporte en plus au moins une chambre d'appoint (100) séparée de ladite au moins une chambre de distribution (40) et de ladite au moins une chambre de collecte (50) par au moins une première paroi séparatrice (60), ladite au moins une chambre d'appoint (100) comprenant un orifice d'entrée (110) d'au moins un fluide d'appoint (F'), distinct des gaz circulant dans ladite au moins une chambre de distribution (40) et dans ladite au moins une chambre de collecte (50), au niveau de la première extrémité (11) et un orifice de sortie (112) dudit au moins un fluide d'appoint (F') au niveau de la deuxième extrémité (12).

2. Réacteur selon la revendication 1, **caractérisé en ce que** chacune desdites au moins une chambre de distribution (40), au moins une chambre de collecte (50) et au moins une chambre d'appoint (100) est délimitée par une section de la paroi interne (21) et deux premières parois séparatrices (60).

3. Réacteur selon la revendication 1 ou 2, **caractérisé en ce qu'**il comporte au niveau de la première extrémité (11) et au niveau de la deuxième extrémité (12), respectivement, un espace distributeur (42) et un espace collecteur (51) entre lesquels l'insert creux (20) est disposé.

4. Réacteur selon l'une des revendications précédentes, **caractérisé en ce que** le tube (10) et l'insert (20) sont maintenus par au moins deux plaques tubulaires de maintien (33) respectivement au niveau des première (11) et deuxième (12) extrémités, au moins l'une desdites au moins deux plaques tubulaires de maintien (33), notamment toutes les plaques tubulaires de maintien (33), comportant un conduit d'entrée ou de sortie (111, 113) respectivement relié fluidiquement à un orifice d'entrée (110) ou de sortie (112) de fluide d'appoint (F') et formé dans ladite au moins l'une desdites au moins deux plaques tubulaires de maintien (33), pour permettre notamment une admission et/ou une extraction latérale de fluide d'appoint (F').

5. Réacteur selon la revendication 4, **caractérisé en ce que** les longueurs axiales du tube (10) et de l'insert (20) sont différentes, la longueur axiale du tube (10) étant notamment plus courte que la longueur axiale de l'insert (20).

6. Réacteur selon la revendication 3, **caractérisé en ce que** le tube (10) et l'insert (20) sont maintenus par au moins deux plaques tubulaires de maintien (33) respectivement au niveau des première (11) et deuxième (12) extrémités, et **en ce qu'**au moins un conduit d'entrée ou de sortie (111, 113) respectivement relié fluidiquement à un orifice d'entrée (110) ou de sortie (112) de fluide d'appoint (F') est situé respectivement dans l'espace distributeur (42) et dans l'espace collecteur (51), hors desdites au moins deux plaques tubulaires de maintien (33), pour permettre notamment une admission et/ou une extraction latérale de fluide d'appoint (F').

7. Réacteur selon la revendication 3, **caractérisé en ce que** le tube (10) et l'insert (20) sont maintenus par au moins deux plaques tubulaires de maintien (33) respectivement au niveau des première (11) et deuxième (12) extrémités, et en ce ledit orifice d'entrée (110) et ledit orifice de sortie (113) dudit au moins un fluide d'appoint (F') sont respectivement formés dans les extrémités supérieure et inférieure de l'insert (20) pour permettre respectivement une alimentation depuis l'espace distributeur (42) et une extraction depuis l'espace collecteur (51), le réacteur (1) comprenant en outre au moins un conduit d'alimentation latéral (140) de gaz et au moins un conduit d'extraction latéral (141) de gaz.

8. Réacteur selon la revendication 7, **caractérisé en ce que** ledit au moins un conduit d'alimentation latéral (140) et l'alimentation en fluide d'appoint (F') de l'espace distributeur (42) d'une part, et ledit au moins un conduit d'extraction latéral (141) et l'extraction en fluide d'appoint (F') de l'espace collecteur (51) d'autre part, sont séparés par une plaque de séparation étanche (105).

9. Réacteur selon la revendication 8, **caractérisé en ce qu'**un joint d'étanchéité (106) est disposé entre l'insert (20) et chacune des plaques de séparation étanches (105).

10. Réacteur selon l'une quelconque des revendications précédentes, **caractérisé en ce que** l'écoulement dudit au moins un fluide d'appoint (F') est réalisé de manière co-courante ou contre-courante à l'écoulement des gaz dans lesdites au moins une chambre de distribution (40) et de collecte (50).

11. Réacteur selon l'une quelconque des revendications précédentes, **caractérisé en ce que** la poudre catalytique est retenue dans l'espace annulaire (30) par un joint (31) en matière fibreuse au niveau de chacune des extrémités de l'espace annulaire (30).

12. Réacteur selon l'une quelconque des revendications précédentes, **caractérisé en ce que** ledit au moins un fluide d'appoint (F') est un fluide caloporteur, notamment un fluide de refroidissement.

13. Réacteur selon l'une quelconque des revendications précédentes, **caractérisé en ce que** l'insert (20) est une pièce monobloc.

14. Utilisation d'un réacteur tubulaire (1) selon l'une quelconque des revendications précédentes, **caractérisée en ce qu'**elle met en œuvre des réactions endothermiques ou exothermiques de synthèse de carburants et de combustibles.

15. Utilisation selon la revendication 14, **caractérisée en ce que** l'espace annulaire (30) permet une réaction d'hydrogénation et **en ce que** ladite au moins une chambre d'appoint (100) permet une réaction de déshydrogénation, ou l'inverse.

# EP 4 412 755 B1

**Patentansprüche**

1.  Rohrreaktor (1) mit Festbett, der sich entlang einer Längsachse (XX') zwischen einem ersten Ende (11) und einem zweiten Ende (12) erstreckt, wobei der Reaktor (1) ein Bett aus Katalysatorpulver umfasst, das in einem Ringraum (30) eingeschlossen ist, der durch eine Außenwand (15) eines Hohlrohrs (10) und eine Innenwand (21) eines Hohleinsatzes (20) begrenzt ist, der koaxial in dem Hohlrohr (10) angeordnet ist, wobei der Hohleinsatz (20) mindestens eine Verteilerkammer (40) und mindestens eine Sammelkammer (50) umfasst, die durch mindestens eine erste Trennwand (60) voneinander getrennt sind, wobei die mindestens eine Verteilerkammer (40) und die mindestens eine Sammelkammer (50) jeweils eine Gaszufuhröffnung (42) an dem ersten Ende (11) und eine Gasablassöffnung (51) an dem zweiten Ende (12) umfassen,
    **dadurch gekennzeichnet, dass** der Reaktor (1) ferner mindestens eine Zusatzkammer (100) aufweist, die von der mindestens einen Verteilerkammer (40) und von der mindestens einen Sammelkammer (50) durch mindestens eine erste Trennwand (60) getrennt ist, wobei die mindestens eine Zusatzkammer (100) eine Einlassöffnung (110) für mindestens ein Zusatzfluid (F'), das sich von den in der mindestens einen Verteilerkammer (40) und in der mindestens einen Sammelkammer (50) zirkulierenden Gasen unterscheidet, an dem ersten Ende (11) und eine Auslassöffnung (112) für das mindestens eine Zusatzfluid (F') an dem zweiten Ende (12) umfasst.

2.  Reaktor nach Anspruch 1, **dadurch gekennzeichnet, dass** jede der mindestens einen Verteilerkammer (40), mindestens einen Sammelkammer (50) und mindestens einen Zusatzkammer (100) durch einen Abschnitt der Innenwand (21) und zwei erste Trennwände (60) begrenzt ist.

3.  Reaktor nach Anspruch 1 oder 2, **dadurch gekennzeichnet, dass** er an dem ersten Ende (11) und an dem zweiten Ende (12) jeweils einen Verteilerraum (42) und einen Sammelraum (51) aufweist, zwischen denen der Hohleinsatz (20) angeordnet ist.

4.  Reaktor nach einem der vorhergehenden Ansprüche, **dadurch gekennzeichnet, dass** das Rohr (10) und der Einsatz (20) durch mindestens zwei Halterohrplatten (33) jeweils an dem ersten (11) und dem zweiten (12) Ende gehalten werden, wobei mindestens eine der mindestens zwei Halterohrplatten (33), insbesondere alle Halterohrplatten (33), einen Einlass- oder einen Auslasskanal (111, 113) aufweisen, der jeweils fluidisch mit einer Einlass- (110) oder einer Auslassöffnung (112) für Zusatzfluid (F') verbunden ist und in der mindestens einen der mindestens zwei Halterohrplatten (33) ausgebildet ist, um insbesondere eine seitliche Zufuhr und/oder eine seitliche Entnahme von Zusatzfluid (F') zu ermöglichen.

5.  Reaktor nach Anspruch 4, **dadurch gekennzeichnet, dass** die axialen Längen des Rohrs (10) und des Einsatzes (20) unterschiedlich sind, wobei die axiale Länge des Rohrs (10) insbesondere kürzer ist als die axiale Länge des Einsatzes (20).

6.  Reaktor nach Anspruch 3, **dadurch gekennzeichnet, dass** das Rohr (10) und der Einsatz (20) durch mindestens zwei Halterohrplatten (33) jeweils an dem ersten (11) und dem zweiten (12) Ende gehalten werden, und dass mindestens ein Einlass- oder ein Auslasskanal (111, 113), der jeweils fluidisch mit einer Einlass-(110) oder einer Auslassöffnung (112) für Zusatzfluid (F') verbunden ist, sich jeweils in dem Verteilerraum (42) und in dem Sammelraum (51) befindet, außerhalb der mindestens zwei Halterohrplatten (33), um insbesondere eine seitliche Zufuhr und/oder eine seitliche Entnahme von Zusatzfluid (F') zu ermöglichen.

7.  Reaktor nach Anspruch 3, **dadurch gekennzeichnet, dass** das Rohr (10) und der Einsatz (20) durch mindestens zwei Halterohrplatten (33) jeweils an dem ersten (11) und dem zweiten (12) Ende gehalten werden, und dass die Einlassöffnung (110) und die Auslassöffnung (113) für das mindestens eine Zusatzfluid (F') jeweils in dem oberen und dem unteren Ende des Einsatzes (20) ausgebildet sind, um eine Versorgung aus dem Verteilerraum (42) und eine Entnahme aus dem Sammelraum (51) zu ermöglichen, wobei der Reaktor (1) ferner mindestens einen seitlichen Gasversorgungskanal (140) und mindestens einen seitlichen Gasentnahmekanal (141) umfasst.

8.  Reaktor nach Anspruch 7, **dadurch gekennzeichnet, dass** der mindestens eine seitliche Versorgungskanal (140) und die Versorgung des Verteilerraums (42) mit Zusatzfluid (F') einerseits und der mindestens eine seitliche Entnahmekanal (141) und die Entnahme von Zusatzfluid (F') aus dem Sammelraum (51) andererseits durch eine wasserdichte Trennplatte (105) voneinander getrennt sind.

9.  Reaktor nach Anspruch 8, **dadurch gekennzeichnet, dass** zwischen dem Einsatz (20) und jeder der wasserdichten Trennplatten (105) eine Dichtung (106) angeordnet ist.

10. Reaktor nach einem der vorhergehenden Ansprüche, **dadurch gekennzeichnet, dass** die Strömung des mindestens einen Zusatzfluids (F') in dem Gleichstrom oder Gegenstrom zur Strömung der Gase in der mindestens einen Verteiler- (40) und einen Sammelkammer (50) erfolgt.

11. Reaktor nach einem der vorhergehenden Ansprüche, **dadurch gekennzeichnet, dass** das Katalysatorpulver in dem Ringraum (30) durch eine Dichtung (31) aus Faserstoff an jedem der Enden des Ringraums (30) zurückgehalten wird.

12. Reaktor nach einem der vorhergehenden Ansprüche, **dadurch gekennzeichnet, dass** das mindestens eine Zusatzfluid (F') ein Wärmeträgerfluid, insbesondere ein Kühlfluid, ist.

13. Reaktor nach einem der vorhergehenden Ansprüche, **dadurch gekennzeichnet, dass** der Einsatz (20) ein einteiliges Bauteil ist.

14. Verwendung eines Rohrreaktors (1) nach einem der vorhergehenden Ansprüche, **dadurch gekennzeichnet, dass** er endotherme oder exotherme Reaktionen zur Synthese von Kraftstoffen und Brennstoffen umsetzt.

15. Verwendung nach Anspruch 14, **dadurch gekennzeichnet, dass** der Ringraum (30) eine Hydrierungsreaktion ermöglicht und dass die mindestens eine Zusatzkammer (100) eine Dehydrierungsreaktion ermöglicht oder umgekehrt.

## Claims

1. A fixed-bed tubular reactor (1) which extends, along a longitudinal axis (XX'), between a first end (11) and a second end (12), said reactor (1) comprising a bed of catalytic powder confined in an annular space (30) delimited by an external wall (15) of a hollow tube (10) and an internal wall (21) of a hollow insert (20) disposed coaxially in the hollow tube (10), the hollow insert (20) comprising at least one distribution chamber (40) and at least one collection chamber (50), separated from each other by at least one first separating wall (60), said at least one distribution chamber (40) and said at least one collection chamber (50) comprising, respectively, a gas admission opening (42) at the first end (11) and a gas evacuation opening (51) at the second end (12),
**characterised in that** the reactor (1) further includes at least one supplemental chamber (100) separated from said at least one distribution chamber (40) and from said at least one collection chamber (50) by at least one first separating wall (60), said at least one supplemental chamber (100) comprising an inlet orifice (110) for at least one supplemental fluid (F'), distinct from the gases circulating in said at least one distribution chamber (40) and in said at least one collection chamber (50), at the first end (11) and an outlet orifice (112) of said at least one supplemental fluid (F') at the second end (12).

2. The reactor according to claim 1, **characterised in that** each of said at least one distribution chamber (40), at least one collection chamber (50) and at least one supplemental chamber (100) is delimited by a section of the internal wall (21) and two first separating walls (60).

3. The reactor according to claim 1 or 2, **characterised in that** it includes at the first end (11) and at the second end (12), respectively, a distributor space (42) and a collector space (51) between which the hollow insert (20) is disposed.

4. The reactor according to one of the preceding claims, **characterised in that** the tube (10) and the insert (20) are held by at least two tubular holding plates (33) respectively at the first (11) and second (12) ends, at least one of said at least two tubular holding plates (33), in particular all the tubular holding plates (33), including an inlet or outlet conduit (111, 113) respectively fluidly connected to a supplemental fluid (F') inlet (110) or outlet (112) orifice and formed in said at least one of said at least two tubular holding plates (33), in particular to allow lateral admission and/or extraction of supplemental fluid (F').

5. The reactor according to claim 4, **characterised in that** the axial lengths of the tube (10) and the insert (20) are different, the axial length of the tube (10) being in particular shorter than the axial length of the insert (20).

6. The reactor according to claim 3, **characterised in that** the tube (10) and the insert (20) are held by at least two tubular holding plates (33) respectively at the first (11) and second (12) ends, and **in that** at least one inlet or outlet conduit (111, 113) respectively fluidly connected to a supplemental fluid (F') inlet (110) or outlet (112) orifice is located respectively in the distributor space (42) and in the collector space (51), outside said at least two tubular holding plates (33), to allow in particular lateral admission and/or extraction of supplemental fluid (F').

7. The reactor according to claim 3, **characterised in that** the tube (10) and the insert (20) are held by at least two tubular holding plates (33) respectively at the first (11) and second (12) ends, and **in that** said inlet orifice (110) and said outlet orifice (113) of said at least one supplemental fluid (F') are respectively formed in the upper and lower ends of the insert (20) to respectively allow a supply from the distributor space (42) and an extraction from the collector space (51), the reactor (1) further comprising at least one lateral gas supply conduit (140) and at least one lateral gas extraction conduit (141).

8. The reactor according to claim 7, **characterised in that** said at least one lateral supply conduit (140) and the supply of supplemental fluid (F') to the distributor space (42) on the one hand, and said at least one lateral extraction conduit (141) and the extraction of supplemental fluid (F') from the collector space (51) on the other hand, are separated by a sealed separation plate (105).

9. The reactor according to claim 8, **characterised in that** a seal (106) is disposed between the insert (20) and each of the sealed separation plates (105).

10. The reactor according to any one of the preceding claims, **characterised in that** the flow of said at least one supplemental fluid (F') is carried out in a co-current or counter-current manner to the flow of gases in said at least one distribution (40) and collection (50) chamber.

11. The reactor according to any one of the preceding claims, **characterised in that** the catalytic powder is retained in the annular space (30) by a seal (31) made of fibrous material at each of the ends of the annular space (30).

12. The reactor according to any one of the preceding claims, **characterised in that** said at least one supplemental fluid (F') is a heat transfer fluid, in particular a cooling fluid.

13. The reactor according to any one of the preceding claims, **characterised in that** the insert (20) is a one-piece part.

14. A use of a tubular reactor (1) according to any one of the preceding claims, **characterised in that** it implements endothermic or exothermic reactions for the synthesis of fuels and combustibles.

15. The use according to claim 14, **characterised in that** the annular space (30) allows a hydrogenation reaction and **in that** said at least one supplemental chamber (100) allows a dehydrogenation reaction, or vice versa.

FIG. 2

FIG. 1

EP 4 412 755 B1

FIG. 3

FIG. 4

FIG. 5

FIG. 6

FIG. 8

FIG. 7

EP 4 412 755 B1

FIG. 10

FIG. 9

EP 4 412 755 B1

FIG. 12

FIG. 11

EP 4 412 755 B1

**EP 4 412 755 B1**

## RÉFÉRENCES CITÉES DANS LA DESCRIPTION

**Documents brevets cités dans la description**

- US 3758279 A
- US 4374094 A
- EP 0560157 A1
- US 2997374 A
- CN 103990420 A
- US 8961909 A
- US 7402719 B2
- FR 3103714 A1